# EUROPEAN PATENT APPLICATION

(11) **EP 0 748 749 A1**
(43) Date of publication of application: **18.12.1996**
(21) Application number: 96304491.2
(22) Date of filing: 17.06.1996
(51) Int. Cl.: B65F 1/00, B65D 5/36, B65D 5/06, A61B 19/02

(54) **Medical waste collection container and blank for forming said container**

(30) Priority: 16.06.1995 JP 174492/95; 30.04.1996 JP 134481/95
(71) Applicant: SANKEMIKAL KABUSHIKIGAISHA, Okazaki-shi, Aichi-ken (JP)
(72) Inventor: Toshio, Kusaka, Anjo-shi, Aichi-ken (JP)
(74) Representative: Greenwood, John David

(57) **Abstract**

A container (1) formable into a box-like form for the collection of medical waste, for example, formed from a rectangular paper material coated on at least one side with a thermoplastic resin. The container (1) is open at one side only and is formed by folding the sheet so that each of a pair of side sheet parts is halved and overlapped in the width direction and by joining the side edges and the bottom edge of the paper material by fusion of the thermoplastic resin.

## Description

The present invention relates to a container that is formed into a box-like form for the collection of medical wastes to be incinerated later, such as various used medical equipment, gauze, chemicals, etc., and a sheet from which such a container is formed.

Various medical wastes, such as the hypodermic needles, syringes, instillation needles, gauze, etc. used in a hospital, etc. are incinerated after being collected in order to prevent secondary infection by viruses, etc. attached to such waste. Due to the ease of incineration, corrugated boxes with a synthetic resin bag attached to the interior are used as containers for such collection.

However, with a container with the above arrangement, a hypodermic needle that is collected may pierce the synthetic resin bag as a result of vibration, etc. during transport or carrying of the container and,in such cases, blood, chemicals, etc. remaining in the bag may leak out to the corrugated box to lower the strength of the box and may become attached to a worker. In the worst case, a hypodermic needle that has pierced through the synthetic resin bag and the corrugated box may directly stick into and injure the worker. Such a container therefore does not always prevent secondary infections effectively.

Although the above disadvantage can be resolved by forming the container from synthetic resin such as foamed polystyrene, etc., such synthetic resin containers cannot be folded and are thus extremely troublesome to transport and store.

Also, since such a container will be incinerated along with the collected medical wastes, it will necessitate high-temperature incineration for preventing the generation of toxic gases during incineration and thus cause the durability of the incineration facility to be lowered. Although this disadvantage can be resolved by incinerating with an incineration facility that can endure high temperatures, this would lead to the cost of the incinerating facility itself to be high.

Furthermore, such a type of medical waste collection container is required to be low in manufacturing cost since it is incinerated upon being used once. However, said cost will be high in the case where the container is a synthetic resin container.

The present invention resolves the above-described disadvantages of the prior art. Embodiments of the invention provide a medical waste collection container. which can reliably prevent the protrusion or leakage of the collected medical wastes to thereby ensure the safety of workers, and which can be manufactured and treated at low cost, are excellent in transportability and storability, and can be made extremely readily into a box-like form upon use. One embodiment of the invention is a sheet for forming such a medical waste collection container.

In order to solve the above-described problems of the prior art, the present invention presents a medical waste collection container formed into a box-like form for the collection of medical wastes, said container comprising a rectangular paper material, which is coated on at least one side with thermoplastic resin and formed into a sealed bag-like form that is opened only on the upper side by folding so that each of a pair of side sheet parts, which form opposing sidewalls of the box-like form, is halved and overlapped on itself in the width direction and joining the side edges and the bottom edge of the paper material by fusion of the thermoplastic resin, and being formable into a box-like form by spreading flat the pair of side sheet parts and folding the corner parts below said side sheet parts in triangular form.

A further aspect of the invention provides a sheet for forming a box-like container for collecting medical waste, that is, a sheet being characterized in being formed from a rectangular paper material, which is coated on at least one side with thermoplastic resin, having first creases, which are provided in the transverse direction so as to respectively demarcate an upper section, which forms the top wall of the box-like form, a middle section, which forms the sidewalls of the box-like form, and a lower section, which forms the bottom wall of the box-like form, second creases, which are provided in the vertical direction so as to demarcate the middle section into the respective side sheet parts, third creases, which are provided in the vertical direction so that each of the pair of mutually opposing side sheet parts may be divided in half and overlapped on itself in the width direction, and fourth creases, which are provided at an inclination of 45 degrees at the lower section below the pair of side sheet parts, and being formable into a sealed bag-like form that is opened only on the upper side by the joining of the side edges and the bottom edge by the fusion of the thermoplastic resin in the condition where the sheet is folded at the third creases.

Further details and advantages of this invention will be seen in the accompanying drawings and following description of preferred exemplary embodiments.

In the drawings:

FIG. 1 is a perspective drawing which shows a medical waste collection container of a first embodiment of the present invention.

FIG. 2 is a front view of the sheet for forming the collection container of FIG. 1.

FIG. 3 is a front view which shows the method by which the sheet of FIG. 2 is formed into the collection container of FIG. 1.

FIG. 4 is a perspective view which shows the method by which the collection container of FIG. 1 is formed into a box-like form.

FIG. 5 is a perspective view which shows the method of use of the collection container of FIG. 4 which has been formed into a box-like form.

FIG. 6 is a perspective view which shows the method of use of the collection container of FIG. 4 which has been formed into a box-like form.

FIG. 7 is a perspective drawing which shows a medical waste collection container of a second embodiment of the present invention.

FIG. 8 is a front view of the sheet for forming the collection container of FIG. 7.

FIG. 9 is a front view which shows the method by which the sheet of FIG. 8 is formed into the collection container of FIG. 7.

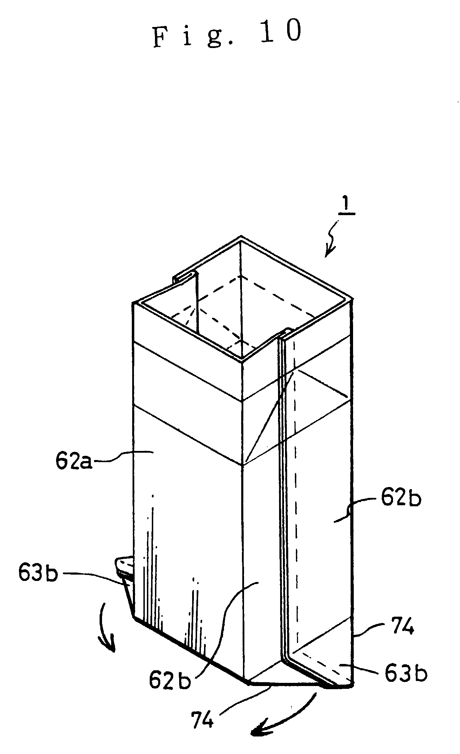

FIG. 10 is a perspective view which shows the method by which the collection container of FIG. 7 is formed into a box-like form.

FIG. 11 is a perspective view which shows the method of use of the collection container of FIG. 10 which has been formed into a box-like form.

FIG. 12 is a perspective view which shows the method of use of the collection container of FIG. 10 which has been formed into a box-like form.

FIG. 13 is a perspective view which shows the method of use of the collection container of FIG. 10 which has been formed into a box-like form.

FIG. 14 is a partial cross section which shows a modified embodiment of the present invention.

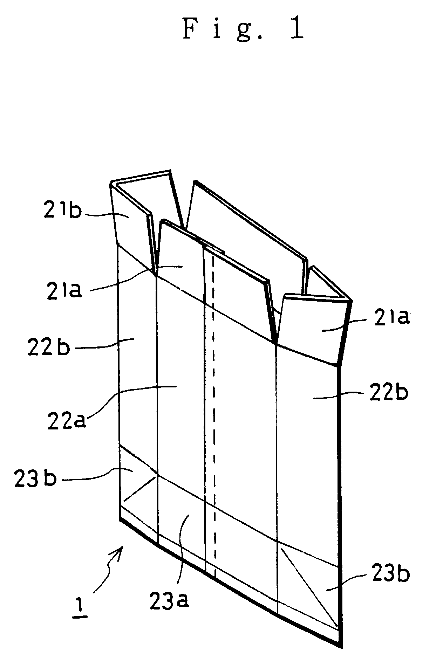

Preferred embodiments of the present invention shall now be described with reference to the drawings. Container 1 in Fig. 1 is formed in a sealed bag-like form which is open only on the upper side and is made from sheet 2 described below.

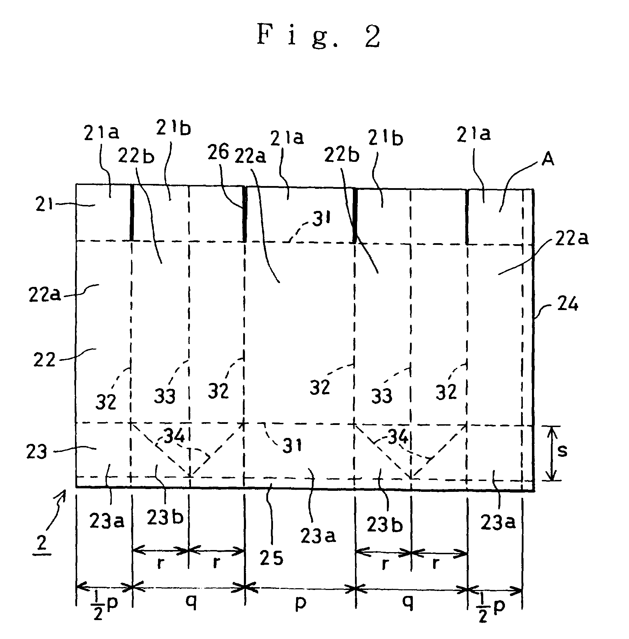

As shown in Fig. 2, this sheet 2 is formed from a rectangular paper material which is coated to a predetermined thickness (preferably about 20-40µm) with thermoplastic resin, such as polypropylene, polyethylene, etc., at least on one side A.

This sheet 2 is formed into a box-like form at the final stage as will be described later and is provided with two first creases 31 that run parallel to each other in the transverse direction so as to demarcate and form an upper section 21, which forms the top wall of the box-like form when the sheet 2 is formed into the box-like form, a middle section 22, which forms the sidewalls, and a lower section 23 which forms the bottom wall and the fold-back part to be described later.

At the middle section 22 are formed one pair each of first side sheet parts 22a and second side sheet parts 22b, which form the opposing sidewalls of the box-like form, and as shown in Fig. 2, four second creases 32, which extend parallel to each other in the vertical direction to the lower section 23, are provided so as to demarcate a first side sheet part 22a with a width p at the center, second side sheet parts 22b, each with a width q, at both sides of said central first side sheet part 22a, and first side sheet parts 22a, each with a width 1/2p, at the outer sides of second side sheet parts 22b. Sheet 2 is also provided with two third creases 33, which extend parallel to each other in the vertical direction from the upper section 21 to the lower section 23 and divide each of the abovementioned pair of second side sheet parts 22b in half in the width direction.

At the lower section 23 are formed a base part 23a, which comprises the bottom wall of the box-like form, and a fold-out part 23b, which is folded outward in a triangular manner so as to form the bottom wall when the box-like form is formed, and, as shown in Fig. 2, the four second creases 32 demarcate a base part 23a with a width p at the center, fold-out parts 23b, each with a width q, at both sides of said central base part 23a, and base parts 23a, each with a width 1/2p, at the outer sides of the fold-out parts 23b.

The vertical dimension s of the lower section 23 is made equal to half the width q of the second side sheet part 22b, in other words, equal to the spacing r between the second crease 32 and third crease 33.

Also at the fold-out part 23b are formed fourth creases 34, which are inclined diagonally downwards at an angle of 45 degrees from the second fold 32 to the third fold 33, and as shall be described later, the fold-out part 23b is made foldable outwards in a triangular manner along the creases 34.

At the upper section 21, notches 26 are provided at four locations in the extensions of the second folds 32 so as to form by parting, a first top sheet part 21a with a width p at the center, second top sheet parts 21b, each with a width q, at both sides of said central first top sheet part 21a, and first top sheet parts 21a, each with a width 1/2p, at the outer sides of second top sheet parts 21b.

Furthermore a first joining edge part 24 and a second joining edge part 25 are respectively formed at the right end edge and bottom end edge of sheet 2.

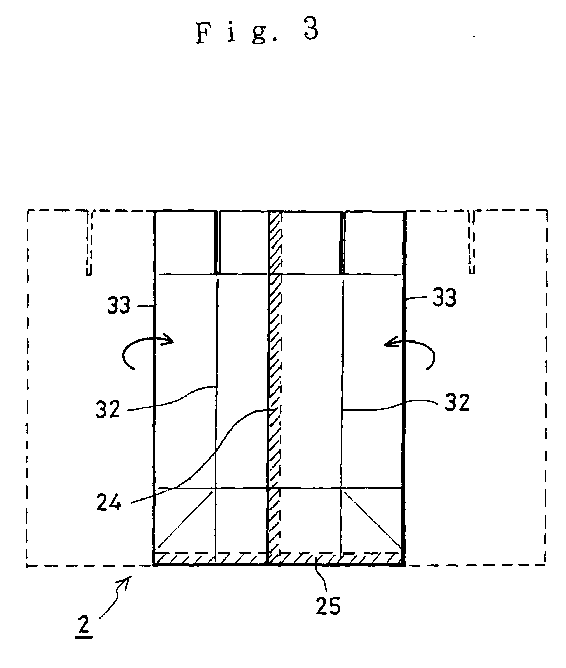

By folding the sheet 2, formed as shown in Fig. 2, by folding inwards at the third crease 33 so that the second side sheet part 22b will be divided in half and overlapped on itself in the width direction and then joining the first joining edge part 24 with the left end edge of the first side sheet part 22a and joining the overlapping parts of the second joining edge part 25 with each other, that is, by respectively joining the locations shown by the slashed parts in Fig. 3, a bag-like container 1, which is opened only on the upper side as shown in Fig. 1, can be made.

In the joining process described above, a strong joint without gaps can be obtained readily by hot pressing the overlapped parts in the condition where the first joining edge part 24 is overlapped with the edge end of the abovementioned first side sheet part 22a and the second joining edge parts 25 are overlapped with each other so as to cause the thermoplastic resin coated on one or both sides to melt to thereby fuse the overlapped parts.

In the case where a sufficient joint strength cannot be secured by only the fusion of the priorly coated thermoplastic resin, a thermoplastic resin film of the same or different type as the thermoplastic coating can be interposed between the abovementioned overlapping parts and joining by fusion can be performed by melting the thermoplastic resin coating and the resin film.

Container 1, which has thus been formed into a bag-like form, is then transported, stored, etc. in the condition where it is folded as shown in Fig. 1 or Fig. 3.

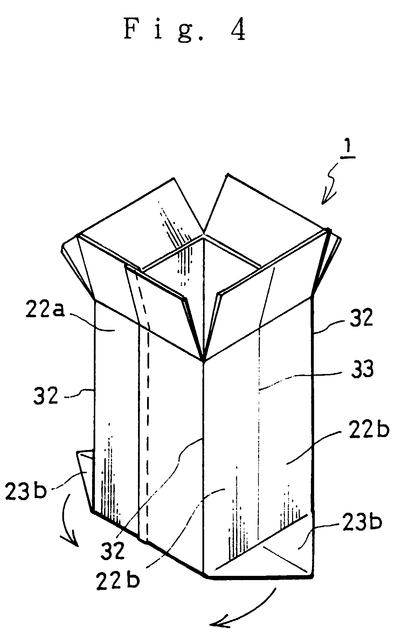

In collecting medical wastes at a hospital, etc., as shown in Fig.4, a box-like form can be formed extremely readily by spreading flat the second side sheet parts 22b, which have been folded in half in the width direction along the third crease 33, folding along the second creases 32 so that the first side sheet part 22a and second side sheet part 22b will be at a right angle with respect to each other, folding out the fold-out parts 23b, disposed at the bottom corner parts of the container 1, into a triangular shape along the fourth creases 34, and spreading flat the base part 23a to form the bottom of container 1 as a flat surface.

The fold-out part 23b, which is folded into a triangular shape, can be folded back into the bottom side to be in close contact with the base part 23a so as not to be a hindrance in using or transporting the container 1. The part of fold-out part 23b which is folded back can be folded back in the upper direction so that the fold-out part 23b will be in close contact with the second side sheet part 22b.

By priorly providing a sealing member, which is covered on the adhesive surface with release paper, at a suitable location of the fold-out part 23b or at the outer wall of the base part 23a or second side sheet part 29b, which comes in contact with the fold-out part 23b, the fold-out part 23b can be put readily into the folded-back condition by the adhesion of the sealing member.

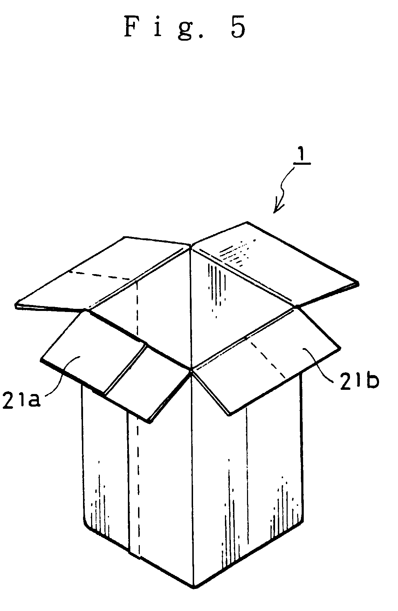

For use, the container 1, which has thus been formed into a box-like form, is set in a condition where it is opened on the upper side by the spreading of the top sheet parts 21a and 21b as shown in Fig. 5 to enable the throwing in of medical wastes from the opening.

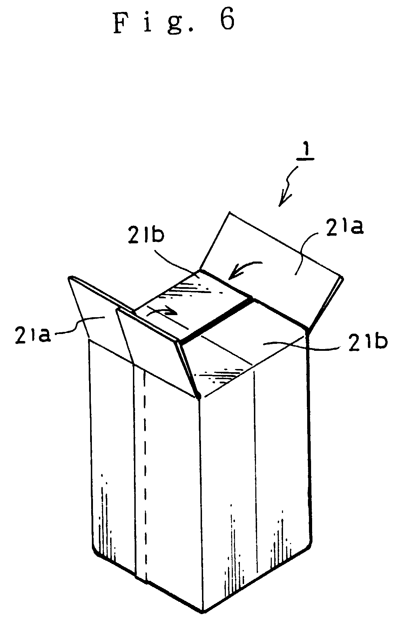

When the amount of medical wastes in the interior of the container reaches a certain amount, the upper opening is closed by folding the top sheet parts 21a and 21b inwards so that they will be mutually overlapped as shown in Fig. 6.

By joining the top sheet parts 21a and 21b by means of adhesive tape, etc. after closing the upper opening or by priorly providing a sealing member, which is covered on the adhesive side with release paper, at suitable locations at which the top sheet parts 21a and 21b are overlapped, the said opening may be closed in an airtight manner and the inadvertent spreading open of top sheet parts 21a and 21b during the transport, etc. of the container 1 can be prevented.

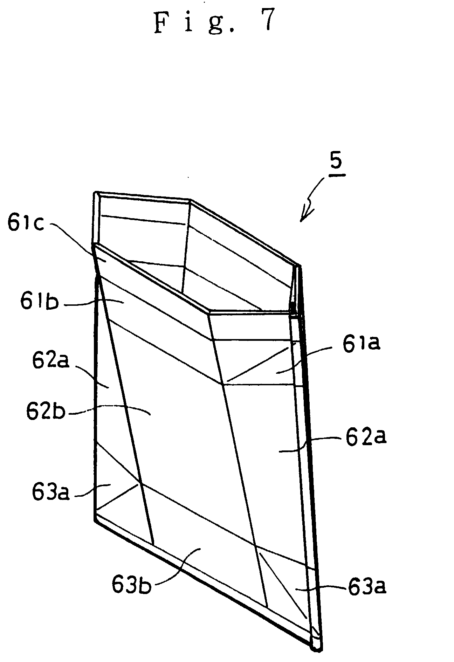

Container 5 in Fig. 7 is formed into a bag-like form which is opened only at the upper side as in the first embodiment and is made from a sheet 6 described below.

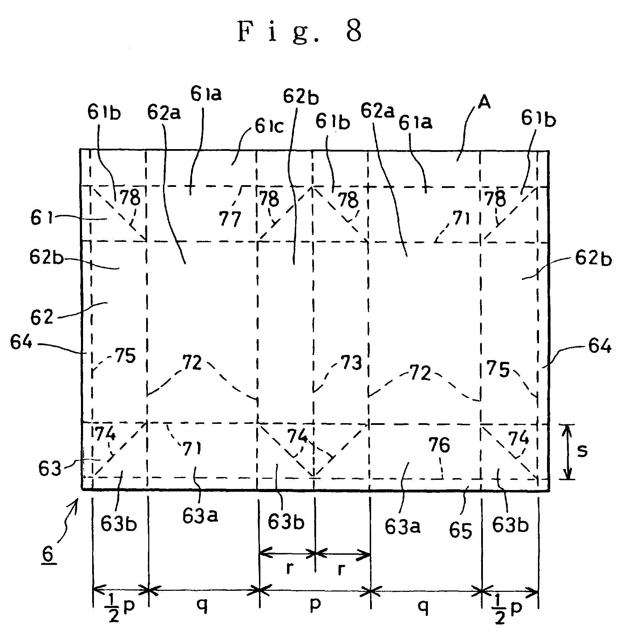

Sheet 6 is formed from the rectangular paper material shown in Fig.8, which, as with the first embodiment, is formed into a box-like form at the final stage and is coated on at least one side A with a thermoplastic resin.

As shown in Fig. 8, the sheet 6 is provided with two first creases 71 that run parallel to each other in the transverse direction so as to respectively demarcate and form an upper section 61, which forms the top wall of the box-like form, a middle section 62, which forms the sidewalls, and a lower section 63 which forms the bottom wall and the fold-out part.

At the middle section 62 are formed one pair each of the first side sheet parts 62a and second side sheet parts 62b, which form the opposing sidewalls of the box-like form and, as shown in Fig. 8, four second creases 72, which extend parallel to each other from the upper section 61 to the lower section 63, are provided in the vertical direction so as to demarcate a second side sheet part 62b with a width p at the center, first side sheet parts 62a, each with a width q, at both sides of said central second side sheet part 62a, and second side sheet parts 62b, each with a width 1/2p, at the outer sides of first side sheet parts 62a.

Sheet 6 is also provided with one third crease 73, which extends in the vertical direction from the upper section 61 to the lower section 63 and which divides the abovementioned central second side sheet part 62b in half in the width direction, and folding sheet 6 can be folded in half along this third crease 73 so that the first side sheet part 62b, provided at the center with a width p, will be overlapped on itself and second side sheet parts 62b of width 1/2p, which are priorly divided in two and provided at the side ends, will be overlapped with each other.

At the lower section 63 are formed a base part 63a, which forms the bottom wall of the box-like form, and a fold-out part 63b, which is folded outward in a triangular manner so as to form said bottom wall when the box-like form is formed, and, as shown in Fig. 8, a fold-out part 63b, with a width p at the center, base parts 63a, each with a width q, at both sides of said central fold-out part 63b, and fold-out parts 63b, each with a width 1/2p, at the outer sides of the base parts 63a, are respectively demarcated by the four second creases 72.

The vertical dimension s of the lower section 63 is made equal to half the width p of the second side sheet part 62b, in other words, equal to the spacing r between the second crease 72 and third crease 73.

Also at the fold-out part 63b are formed fourth creases 74, which are diagonally inclined downwards at an angle of 45 degrees from the second crease 72 to the third crease 73 and from the second crease 72 to both bottom corners of the sheet, and the fold-out part 63b is made foldable outwards in a triangular manner along the creases 74 when the sheet 6 is formed into a box-like form.

Sheet 6 is also provided with the fifth creases 75 and sixth crease 78 which demarcate and form first joining edge parts 64 at both side edges of sheet 6 and a second joining part 65 at the bottom end edge of sheet 2.

At the upper section 61 are formed a top sheet part 61a, which forms the top wall of the box-like form, and a fold-in part 61b, which is folded inwards when the top wall is arranged with the top sheet part 61a, and, as shown in Fig. 8, a first fold-in part 61b with a width p at the center, top sheet parts 61a, each with a width q, at both sides of said central fold-in part 61b, and fold-in parts 61b, each with a width 1/2p, at the outer sides of top sheet parts 61a, are respectively demarcated by the four second creases 72.

Sheet 6 is also provided with a seventh crease 77, which extends in the transverse direction above the top sheet part 61a and fold-in part 61b, that is, at the top end edge of sheet 6 so as to form a fold back edge part 61c, which is folded back in the upward direction when the top wall of the box-like form is formed with the top sheet part 61a.

And, at each fold-in part 61b, are formed the eighth creases 76, which are diagonally inclined upwards at an angle of 45 degrees from the second crease 72 to the third crease 73 and from the second crease 72 to the point of intersection of the fifth crease 75 and seventh crease 77.

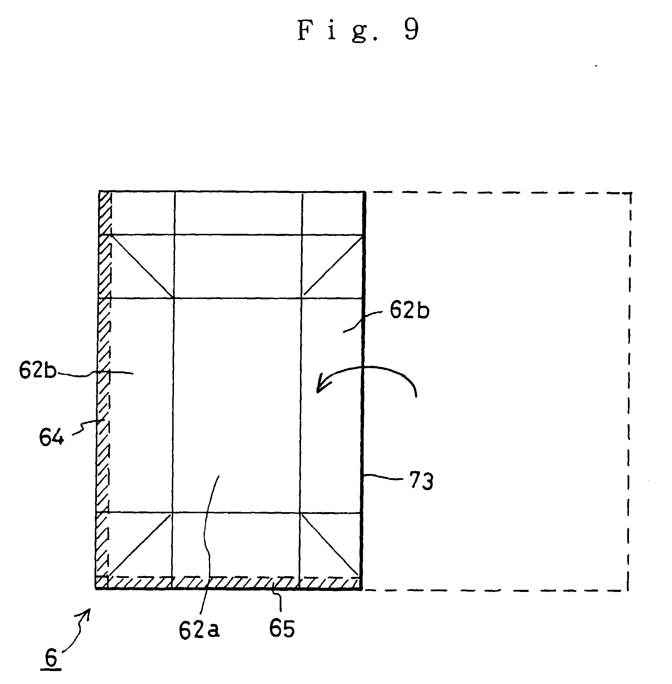

By folding the sheet 6, formed as shown in Fig. 8, in half at the third crease 73 so that the second side sheet part 62b will be divided in half and overlapped on itself in the width direction and then joining the overlapped side ends of the first joining edge part 64 with each other and the second joining edge part 65 with each other, that is, by respectively joining the locations shown by the slashed parts in Fig. 9, the bag-like container 5, which is opened only on the upper side as shown in Fig. 7, can be made.

The joining process described above can be performed as in the first embodiment by hot pressing the mutually overlapped parts of the first joining edge part 64 and the mutually overlapped parts of the second joining edge part 65 so as to melt the thermoplastic resin coated on one or both sides of the overlapped parts.

Container 5, which has thus been formed into a bag-like form, is then transported, stored, etc. in the condition where it is folded as shown in Fig. 7 or Fig. 9.

And in collecting medical wastes at a hospital, etc., a box-like form can be formed extremely readily by spreading flat the second side sheet parts 62b, which have been folded in half in the width direction, folding along the second creases 72 so that the first side sheet part 62a and second side sheet part 62b will be at a right angle with respect to each other, folding out the fold-out parts 63b, disposed at the bottom corner parts of container 5, in a triangular manner along the fourth creases 74, and spreading flat the base part 63a to thereby form the bottom of container 5 as a flat surface.

As in the first embodiment, the fold-out part 63b, which is folded into a triangular manner, is preferably folded back to be in close contact with the base part 63a or second side sheet part 62b so as not to be a hindrance in using or transporting the container 5.

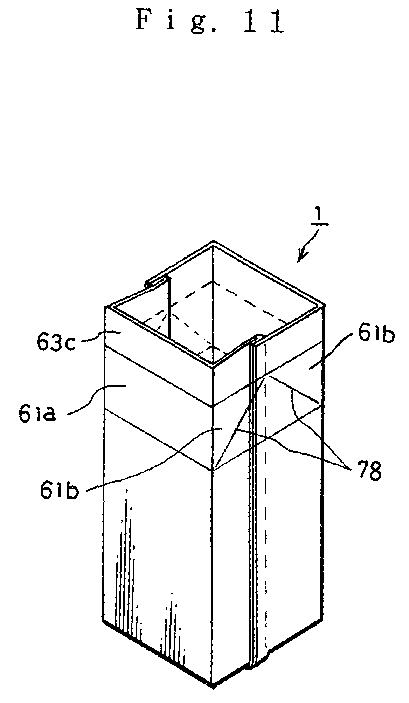

Container 5, which has thus been formed into a box-like form, is set upon use with the top sheet parts 61a and fold-in parts 61b upright so that the upper side will be open as shown in Fig. 11 to enable the throwing in of medical wastes from the opening.

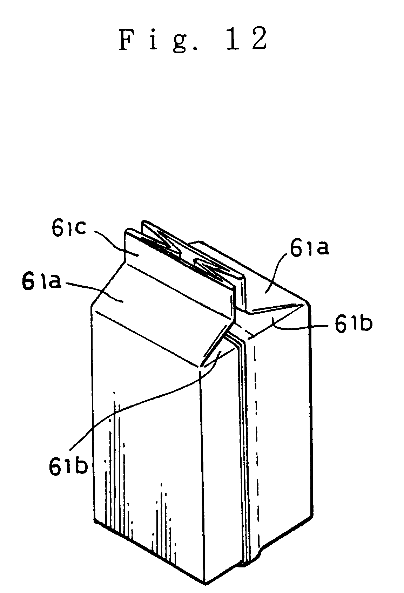

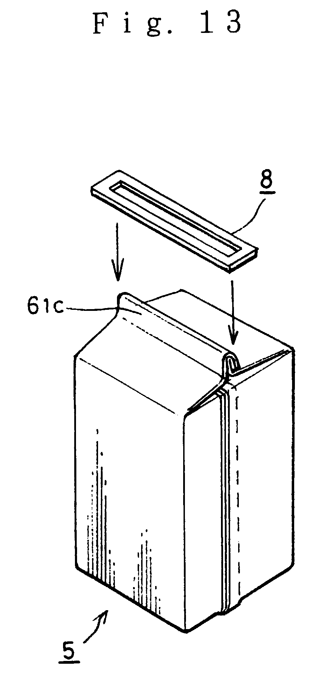

When the amount of medical wastes in the interior of the container reaches a certain amount, fold-in parts 61b are folded inwards in a triangular manner along the eighth creases 76 and top sheet parts 61c are folded inward as shown in Fig. 12 to close the opening. In this process, the overlapped fold-in edge parts 61c are raised upward and then folded back and this folded-back condition is maintained by fitting a fixing member 8 onto the fold-in edge parts 61c as shown in Fig. 13. By priorly providing a sealing member at the overlapped parts of the fold-in edge parts 61c, the upper opening of container 5 formed into a box-like form may be closed in an airtight manner to prevent the leakage of blood, chemicals, etc. in the interior.

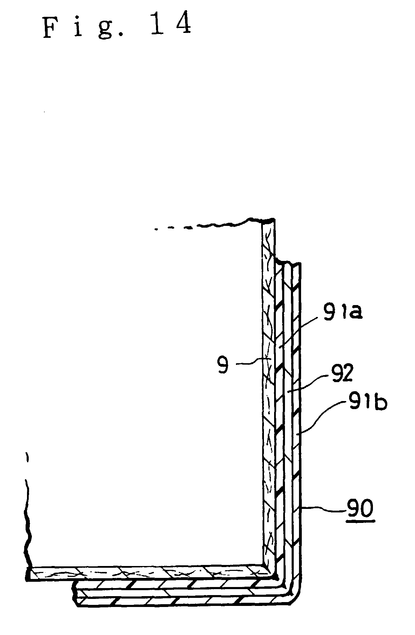

Although the collection of ordinary medical wastes has been described with regard to the above embodiments, the present invention may also be used for the collection, for example, of medical wastes such as chemicals containing a radioactive substance or equipment, etc. with which such chemicals were used. In this case, the sheet and the collection container may be formed by fusing a shielding film 90, in which a lead layer 92 is laminated between resin films 91a and 91b, over the entire outer surface of paper material 9 as shown in Fig. 14.
In this case, the emission to the exterior of the radioactivity of the radioactive substance contained in the collected chemical, etc. is prevented by the lead layer 92 of the fused shielding film 90 to ensure the safety of the worker.

Also with the present invention, the sheet can be made extremely inexpensively by using, for example, cutting wastage discharged in the production of paper beverage containers which are coated on one side with thermoplastic resin or wastepaper materials including defective products which cannot be used as a product due to there being a pinhole at part of the thermoplastic resin layer coated on said paper container.

Since such wastepaper material are usually unmatched in dimension, they can be placed flat and cut into rectangular sheets of the desired size, that is, of a size that corresponds to the volume of the medical wastes to be contained.

Also with such wastepaper material, since thermoplastic resin is coated on one side in advance, sheets of the desired waterproofness and resistance against piercing can be made readily by hot pressing at least two sheets of wastepaper material in the layered condition and fusing the sheets mutually by the melting of the thermoplastic resin. The number of sheets to be layered may be set to three or more in accordance with the required waterproofness and resistance against piercing.

Furthermore, if in the mutual fusion of the abovementioned wastepaper material, sufficient fusion strength cannot be obtained with only the coated thermoplastic resin layer, fusion can be performed upon layering a thermoplastic resin film of the same or different type as the thermoplastic resin coated mutually between the wastepaper materials and then melting the thermoplastic resin coating and film.

The invention has been described in an illustrative manner, and it is to be understood that the terminology which has been used is intended to be in the nature of words of description rather than of limitation.

Obviously, many modifications and variations of the present invention are possible in light of the above teachings. It is, therefore, to be understood that within the scope of the appended claims wherein reference numerals are merely for convenience and are not to be in any way limiting, the invention may be practiced otherwise than as specifically described.

## Claims

1. A medical waste collection container formed into a box-like form for the collection of medical wastes, said container comprising:
a rectangular paper material, which is coated on at least one side with thermoplastic resin and formed into a sealed bag-like form that is opened only on the upper side by folding so that each of a pair of side sheet parts, which form opposing sidewalls of the box-like form, is halved and overlapped on itself in the width direction and joining the side edges and the bottom edge of the paper material by fusion of the thermoplastic resin,
and being formable into a box-like form by spreading flat the pair of side sheet parts and folding the corner parts below said side sheet parts in triangular form.

2. A medical waste collection container as set forth in claim 1, wherein a top sheet part, which closes the upper opening when the container is formed into a box-like form, is formed above the side sheet part.

3. A sheet for forming a box-like container for collecting medical waste, said sheet being characterized in being formed from a rectangular paper material, which is coated on at least one side with thermoplastic resin,
having first creases, which are provided in the transverse direction so as to respectively demarcate an upper section, which forms the top wall of the box-like form, a middle section, which forms the sidewalls of the box-like form, and a lower section, which forms the bottom wall of the box-like form, second creases, which are provided in the vertical direction so as to demarcate the middle section into the respective side sheet parts, third creases, which are provided in the vertical direction so that each of the pair of mutually opposing side sheet parts may be divided in half and overlapped on itself in the width direction, and fourth creases, which are provided at an inclination of 45 degrees at the lower section below the pair of side sheet parts,
and being formable into a sealed bag-like form that is opened only on the upper side by the joining of the side edges and the bottom edge by the fusion of the thermoplastic resin in the condition where the sheet is folded at the third creases.
